Europäisches Patentamt

(19) European Patent Office

Office européen des brevets

(11) EP 0 488 840 B1

## (12) FASCICULE DE BREVET EUROPEEN

(45) Date de publication et mention
de la délivrance du brevet:
**17.01.1996 Bulletin 1996/03**

(51) Int. Cl.⁶: **A61N 1/368**

(21) Numéro de dépôt: **91403008.5**

(22) Date de dépôt: **08.11.1991**

(54) **Procédé de contrôle du rythme cardiaque d'un porteur de stimulateur cardiaque double chambre**

Verfahren zur Steuerung des Herzrhythmus eines Trägers von einem Zweikammer-Herzschrittmacher

Method for controlling the cardiac rhythm of a bearer of a dual-chamber pacemaker

(84) Etats contractants désignés:
**BE CH DE ES GB IT LI SE**

(30) Priorité: **30.11.1990 FR 9015011**

(43) Date de publication de la demande:
**03.06.1992 Bulletin 1992/23**

(73) Titulaire: **ELA-MEDICAL (Société Anonyme)**
**F-92541 Montrouge (FR)**

(72) Inventeurs:
• **Girodo, Sylvie**
**F-92120 Montrouge (FR)**

• **Malherbe, Odile**
**F-94230 Cachan (FR)**
• **Limousin, Marcel**
**F-92120 Montrouge (FR)**

(74) Mandataire: **Laget, Jean-Loup et al**
**F-75116 Paris (FR)**

(56) Documents cités:
EP-A- 0 318 304    US-A- 4 313 442
US-A- 4 515 161    US-A- 4 890 617

## Description

La présente invention concerne un procédé de contrôle du rythme cardiaque d'un patient, porteur d'un stimulateur double chambre tendant à éviter une augmentation exagérée du rythme cardiaque, en cas d'extra-systole auriculaire isolée, comme en cas de tachycardie auriculaire installée.

De manière connue, on met en mémoire dans le stimulateur cardiaque destiné au contrôle du coeur, deux valeurs de fréquence de stimulation du ventricule.

L'une de ces valeurs est la fréquence de base ou fréquence minimale de stimulation, et l'autre est la fréquence maximale au delà de laquelle il est dangereux de stimuler le ventricule.

Lorsque le stimulateur détecte une augmentation de l'activité auriculaire, il stimule le ventricule à une fréquence approchant celle de l'oreillette, sans toutefois dépasser la fréquence maximale imposée.

Un premier problème qui se pose dans les stimulateurs cardiaques de type connu est la difficulté à distinguer une accélération physiologique du rythme auriculaire, d'une accélération pathologique.

Un second problème qui se pose lors d'une accélération de l'oreillette au delà de la fréquence maximale est d'apprécier la durée pendant laquelle le coeur est stimulé à une fréquence rapide avant de disposer du mode de repli vers une fréquence de base.

Le troisième problème au cours de cette procédure de repli est la dissociation entre les dépolarisations à l'étage auriculaire et les dépolarisations à l'étage ventriculaire.

Dans les stimulateurs cardiaques existants, ces problèmes sont résolus de manière imparfaite. Dans le document US 4.467.810, par exemple, la procédure de stimulation en mode 2/1 intervient dès que la fréquence de dépolarisation de l'oreillette atteint la fréquence de référence, et les dépolarisations de l'oreillette qui surviennent à une fréquence supérieure à une fréquence de référence programmée, sont systématiquement ignorées.

En outre, le stimulateur dispose d'une procédure de repli, c'est-à-dire de ralentissement du rythme de stimulation du ventricule indépendamment du rythme de l'oreillette.

Afin d'éviter ces différents inconvénients, l'invention a pour but d'identifier le type d'accélération auriculaire, de ralentir le rythme ventriculaire en cas de dépassement de la fréquence maximale programmée, en dissociant l'oreillette et le ventricule lorsqu'une accélération anormale de l'oreillette est détectée, et de conserver une association auriculo-ventriculaire dans les autres cas, dans la limite de la fréquence maximale de stimulation ventriculaire.

L'invention a pour objet un procédé de contrôle du rythme cardiaque d'un porteur d'un stimulateur double chambre caractérisé en ce qu'on définit une période réfractaire auriculaire post auriculaire (PRAPA) durant laquelle toute dépolarisation de l'oreillette est définie comme pathologique et on ne déclenche pas, à sa détection, le délai auriculo-ventriculaire.

Selon d'autres caractéristiques de l'invention :

- la PRAPA est égale à une fraction comprise entre 0,7 et 0,8 fois, et de préférence égale à 0,75 fois le temps séparant deux ondes "P" précédentes physiologiques ;

- la PRAPA est définie comme la moyenne glissante sur les huit derniers événements auriculaires, en cas de présence d'extra-systole auriculaire ou ventriculaire ;

- la PRAPA a une durée maximale comprise entre 400 et 600ms et de préférence égale à 560ms ;

- à la détection d'une onde "P" survenant au cours de la PRAPA, on déclenche un intervalle d'échappement auriculaire de durée égale à la PRAPA et, en l'absence de détection d'une onde "P" ultérieure, on stimule l'oreillette à la fin du délai le plus long entre ce dernier intervalle et l'intervalle d'échappement auriculaire déclenché lors de l'onde "P" précédente ;

- en cas de détection d'une onde "P" hors PRAPA, ou en cas de stimulation de l'oreillette, on déclenche un délai auriculo-ventriculaire de durée minimale ;

- à la fin dudit délai auriculo-ventriculaire minimal, le ventricule est stimulé si le temps écoulé depuis sa dernière stimulation permet de respecter la fréquence maximale de stimulation ;

- si, à la fin dudit délai auriculo-ventriculaire minimal, on doit prolonger celui-ci afin de respecter la fréquence maximale de stimulation, alors on déclenche, après la stimulation du ventricule, une PRAPA dont la durée est égale au temps devant séparer deux stimulations à la fréquence maximale, réduit de la valeur du délai auriculo-ventriculaire minimal ;

- après avoir observé l'installation de la tachycardie pendant un temps défini, de préférence égal à 30 secondes, on déclenche le repli consistant à faire baisser de manière arbitraire la fréquence de stimulation du ventricule jusqu'à une fréquence stable fixée à 70 c/mn par exemple ;

- lorsque, au cours de la phase de repli, l'intervalle minimal sur huit cycles entre deux ondes "P" consécutives devient supérieur à la période minimale Pmin correspondant à la fréquence maximale de stimulation progammée pour le ventricule, on sort de la phase de repli et on revient au mode initial de stimulation de coeur.

D'autres caractéristiques ressortent de la description qui suit faite avec référence au dessin annexé sur lequel on peut voir :

Figure 1 : un schéma de mise en oeuvre du procédé de contrôle du rythme cardiaque selon l'invention dans le cas d'une extra-systole auriculaire isolée ;

Figure 2 : un schéma de mise en oeuvre du procédé selon l'invention dans un exemple de tachycardie auriculaire de fréquence inférieure à deux fois la fréquence maximale de stimulation ;

Figure 3 : un schéma de mise en oeuvre du procédé selon l'invention dans un exemple de tachycardie auriculaire de fréquence supérieure à deux fois la fréquence maximale de stimulation.

Le procédé de contrôle du rythme cardiaque suivant l'invention, se différencie des procédés usuels en ce que l'on détecte de manière effective une accélération de l'activité de l'oreillette afin de l'utiliser comme un nouveau paramètre physiologique, et de s'assurer que l'on est effectivement en présence d'une accélération pathologique.

A cet effet, on modifie tout d'abord les périodes réfractaires absolues auriculaire et ventriculaire.

De manière usuelle, après chaque dépolarisation du ventricule, on déclenche pour l'oreillette et le ventricule des périodes réfractaires absolues durant lesquelles on ne sait pas ce qui se passe dans l'oreillette et on ne veut pas stimuler le ventricule. Ces périodes habituellement égales sont, suivant l'invention, réduites à leur durée minimale nécessaire. La période réfractaire absolue ventriculaire varie peu par rapport aux valeurs usuelles, de l'ordre de 204 à 350ms (millisecondes), mais la période réfractaire absolue auriculaire, est limitée au temps de repos nécessaire aux amplificateurs, soit environ 110ms. De cette manière, on peut examiner et prendre en compte ce qui se passe au niveau de l'oreillette, beaucoup plus rapidement et durant 94 à 240ms supplémentaires à chaque cycle.

La période réfractaire absolue auriculaire est de cette manière réduite à une valeur telle que l'on peut observer avec précision une accélération du rythme auriculaire et déterminer si cette accélération est pathologique ou physiologique.

Dans le procédé de contrôle du rythme cardiaque selon l'invention, on définit une période durant laquelle, on peut définir comme pathologique toute dépolarisation détectée de l'oreillette.

Cette période, que l'on nomme période réfractaire auriculaire post auriculaire (PRAPA), est définie comme ayant une durée égale à 0,7 à 0,8 fois le temps séparant deux ondes "P", cette durée devant toujours être inférieure à une valeur déterminée entre 400 et 600ms.

De préférence, la PRAPA est égale à 0,75 fois le temps séparant deux ondes "P", et toujours inférieure à 560ms. Ces valeurs sont telles que l'on est certain de ne pas prendre en compte des accélérations physiologiques, car celles-ci ne dépassent pas ces valeurs de façon soutenue : seules les accélérations pathologiques donnent d'assez grandes variations pour dépasser ces valeurs.

La durée séparant deux ondes "P" est mesurée entre deux ondes "P", dites compétentes, c'est-à-dire entre des dépolarisations de l'oreillette ayant déclenché un délai auriculo-ventriculaire (DAV).

Le délai entre deux ondes "P" utilisé lors de la détection d'une dépolarisation de l'oreillette est, lorsque le cycle précédent était normal, le délai effectivement mesuré; mais il est, lorsque le cycle précédent était pathologique, la moyenne des valeurs mesurées au cours des huit derniers cycles normaux.

Au cours de la PRAPA déclenchée à la suite d'une dépolarisation de l'oreillette, on observe celle-ci :

- si la première onde "P" suivante arrive après la PRAPA, alors l'oreillette n'a pas subi une accélération supérieure à 25% et on déclenche de la manière habituelle un délai auriculo-ventriculaire,

- si une onde "P" est détectée au cours de la PRAPA, on ne déclenche pas de délai auriculo-ventriculaire car on considère que l'on a affaire à une extra-systole.

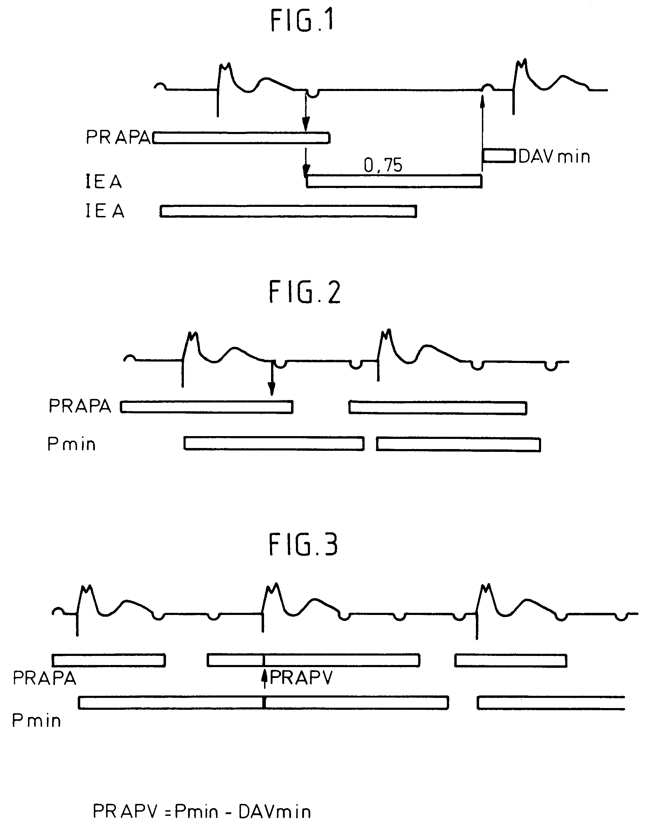

A la détection de cette onde "P" au cours de la PRAPA, on déclenche un intervalle d'échappement auriculaire (IEA) de durée égale à la PRAPA (Fig. 1).

L'intervalle d'échappement auriculaire est, de manière connue en soi, le temps au bout duquel il faut stimuler l'oreillette si elle ne s'est pas dépolarisée spontanément.

A la fin du délai le plus long, c'est-à-dire soit à la fin de l'intervalle d'échappement auriculaire suivant l'onde "P" détectée au cours de la PRAPA lancée à partir de la première onde "P", soit à la fin de l'intervalle d'échappement auriculaire déclenché à la première onde "P", on stimule l'oreillette puis on déclenche un délai auriculo-ventriculaire égal au délai minimal programmé (Figure 1).

Le délai auriculo-ventriculaire minimal est choisi afin de ne pas prolonger de manière trop importante le cycle ventriculaire.

A la fin dudit délai auriculo-ventriculaire, le ventricule est stimulé si le délai (Pmin), écoulé depuis la dernière stimulation de celui-ci permet de respecter la fréquence maximale de stimulation.

Ainsi, le ventricule est alors stimulé une seule fois tandis que l'oreillette s'est dépolarisée deux fois. On a alors reproduit une association dite 2/1 afin de ne pas accélérer le ventricule (Figure 2).

Dans le cas de Fig. 3, la tachycardie auriculaire est de fréquence supérieure à deux fois la fréquence maximale de stimulation. La première onde "P" hors PRAPA déclenche une PRAPA et un DAV qui se termine avant la fin de la période minimale Pmin. Le DAV est donc prolongé jusqu'à la fin de la période Pmin. A la fin de cette période Pmin, on stimule le ventricule et on substitue à la PRAPA déclenchée par l'onde "P", une période réfractaire auriculaire post ventriculaire PRAPV dont la durée est égale à la période minimale Pmin diminuée du DAV minimal :

PRAPV = Pmin - DAV min = Pmin - 31 ms.

La première onde "P" détectée après cette PRAPV déclenche un DAV minimal de 31 ms.

Si la tachycardie est installée, on a alors une stimulation en 3/1 pour laquelle il y a une stimulation du ventricule pour trois détections de l'oreillette. Ce cycle 3/1 alternera avec des cycles 2/1.

L'utilisation de ce principe de la PRAPA et de son mode de fonctionnement permet de faire passer le rythme ventriculaire en mode (2/1, 3/1), ou éventuellement en mode (n/1, (n+1)/1), lors de troubles du rythme auriculaire, et ainsi de ne pas augmenter inutilement la fréquence de stimulation du ventricule.

Si la situation décrite au-dessus se pérennise, on constate que l'on est en présence d'une tachycardie pathologique.

Au bout d'un délai que l'on peut par exemple fixer à 30 secondes, on déclenche la phase dite de repli au cours de laquelle on dissocie le ventricule de l'oreillette, et on fait baisser de manière arbitraire la fréquence de stimulation du ventricule jusqu'à une fréquence stable fixée par exemple à 70 battements par minute.

Ce repli se fait suivant le mode usuellement nommé VVI, ce qui signifie que la détection d'une dépolarisation du ventricule inhibe la stimulation qu'il devait recevoir.

Au cours de la phase de repli, on observe l'intervalle PP entre deux ondes "P" consécutives. Lorsque l'intervalle PP minimal sur 8 cycles devient supérieur à la période minimale Pmin correspondant à la fréquence maximale de stimulation programmée pour le ventricule, on sort de la phase de repli et on revient au mode initial de stimulation du coeur.

L'instauration d'un délai de 30 secondes avant le déclenchement du repli permet de ne déclencher celui-ci que sur des tachycardies soutenues.

Un tel procédé permet alors de ne jamais générer un rythme ventriculaire rapide lors d'une tachycardie, quelle que soit sa durée.

## Revendications

1. Procédé de commande d'un stimulateur cardiaque double chambre, caractérisé en ce que l'on définit une période réfractaire auriculaire post auriculaire (PRAPA) durant laquelle toute dépolarisation de l'oreillette est définie comme pathologique et on ne déclenche pas de délai auriculo-ventriculaire en réponse à cette dépolarisation.

2. Procédé selon la revendication 1, caractérisé en ce que la PRAPA est égale à une fraction comprise entre 0,7 et 0,8 fois, et de préférence égale à 0,75 fois le temps séparant deux ondes "P" physiologiques.

3. Procédé selon la revendication 1, caractérisé en ce que la PRAPA est égale à une fraction comprise entre 0,7 et 0,8 fois de préférence égale à 0,75 fois la moyenne des huit derniers événements auriculaires en cas de présence d'extra-systole auriculaire ou ventriculaire.

4. Procédé selon la revendication 1, caractérisé en ce que la PRAPA a une durée maximale comprise entre 400 et 600ms et de préférence égale à 560ms.

5. Procédé selon la revendication 1, caractérisé en ce que, à la détection d'une onde "P" survenant au cours de la PRAPA, on déclenche un intervalle d'échappement auriculaire de durée égale à la PRAPA et, en l'absence de détection d'une onde "P" ultérieure, on déclenche une stimulation auriculaire à la fin du délai le plus long entre ce dernier intervalle et l'intervalle d'échappement auriculaire déclenché lors de l'onde "P" précédente.

6. Procédé selon la revendication 5 caractérisé en ce que, en cas de détection d'une onde "P" hors PRAPA, ou en cas de stimulation auriculaire, on déclenche un délai auriculo-ventriculaire de durée minimale.

7. Procédé selon la revendication 5, caractérisé en ce que, à la fin dudit délai auriculo-ventriculaire minimal, on déclenche une stimulation ventriculaire si le temps écoulé depuis sa dernière stimulation permet de respecter la fréquence maximale de stimulation.

8. Procédé selon la revendication 5, caractérisé en ce que si, à la fin dudit délai auriculo-ventriculaire minimal, on doit prolonger celui-ci afin de respecter la fréquence maximale de stimulation, alors on déclenche, après la stimulation ventriculaire, une PRAPV dont la durée est égale au temps devant séparer deux stimulations à la fréquence maximale, réduit de la valeur du délai auriculo-ventriculaire minimal.

9. Procédé selon la revendication 7 ou la revendication 8, caractérisé en ce que, après avoir observé l'installation de la tachycardie pendant un temps défini, de préférence égal à 30 secondes, on déclenche le repli consistant à faire baisser de manière arbitraire la fréquence de stimulation ventriculaire jusqu'à une fréquence stable fixée à 70c/mn par exemple.

10. Procédé selon la revendication 9, caractérisé en ce que, lorsque, au cours de la phase de repli, l'intervalle minimal sur huit cycles entre deux ondes "P" consécutives devient supérieur à la période minimale Pmin correspondant à la fréquence maximale de stimulation ventriculaire progammée, on sort de la phase de repli et on revient au mode initial de stimulation du coeur.

## Claims

1. A method of controlling a dual-chamber cardiac pacemaker, characterised in that a post-atrial atrial refractory period (PRAPA) is defined, during which any depolarisation of the atrium is defined as a pathology and no atrioventricular delay is triggered in response to this depolarisation.

2. A method according to Claim 1, characterised in that the PRAPA is equal to a fraction of between 0.7 and 0.8 times, and preferably equal to 0.75 times, the time between two physiological P waves.

3. A method according to Claim 1, characterised in that the PRAPA is equal to a fraction of between 0.7 and 0.8 times, and preferably equal to 0.75 times, the average of the last eight atrial events in the event of the presence of an atrial or ventricular extrasystole.

4. A method according to Claim 1, characterised in that the PRAPA has a maximum duration of between 400 and 600 ms, and preferably equal to 560 ms.

5. A method according to Claim 1, characterised in that upon detection of a P wave occurring during the PRAPA an atrial escape interval of a duration equal to the PRAPA is triggered and, in the absence of detection of a subsequent P wave atrial pacing is triggered at the end of the longest delay between said last interval and the atrial escape interval triggered upon the preceding P wave.

6. A method according to Claim 5, characterised in that in the event of detection of a P wave outside PRAPA, or in the event of atrial stimulation, an atrioventricular delay of minimum duration is triggered.

7. A method according to Claim 5, characterised in that at the end of said minimum atrioventricular delay ventricular pacing is triggered if the time elapsed since the last stimulation makes it possible to respect the maximum stimulation frequency.

8. A method according to Claim 5, characterised in that if, at the end of said minimum atrioventricular delay, it is necessary to prolong the latter in order to respect the maximum stimulation frequency, then a PRAPV, the duration of which is equal to the time which is supposed to separate two stimulations at maximum frequency, reduced by the value of the minimum atrioventricular delay, is triggered after the ventricular pacing.

9. A method according to Claim 7 or Claim 8, characterised in that, after having observed the onset of the tachycardia for a defined time, preferably 30 seconds, the fallback consisting in arbitrarily lowering the ventricular stimulation frequency to a stable frequency fixed at 70 c/mn for example is triggered.

10. A method according to Claim 9, characterised in that when, during the fallback phase, the minimum interval over eight cycles between two consecutive P waves becomes greater than the minimum period Pmin corresponding to the maximum programmed ventricular stimulation frequency, the fallback phase is exited and a return is effected to the initial mode of stimulation of the heart.

## Patentansprüche

1. Verfahren zur Steuerung eines Zwei-Kammer-Herzschrittmachers,
**dadurch gekennzeichnet, daß**
eine aurikulär-postaurikuläre Refraktärphase (PRAPA) definiert wird, während der jede Depolarisation des Herzohres als pathologisch definiert ist und keine aurikulär-ventrikuläre Verzögerung im Ansprechen auf diese Depolarisation ausgelöst wird.

2. Verfahren gemäß Patentanspruch 1,
**dadurch gekennzeichnet, daß**
die PRAPA gleich einem zwischen dem 0,7- und 0,8-fachen liegenden und vorzugsweise 0,75 betragenden Bruchteil der zwei physiologische "P"-Zacken trennenden Zeit ist.

3. Verfahren gemäß Patentanspruch 1,
**dadurch gekennzeichnet, daß**
die PRAPA im Fall des Vorliegens einer aurikulären oder ventrikulären Extrasystole gleich einem zwischen dem 0,7- und 0,8-fachen liegenden und vorzugsweise 0,75 betragenden Bruchteil des Mittelwerts der acht letzten aurikulären Ereignisse ist.

4. Verfahren gemäß Patentanspruch 1,
**dadurch gekennzeichnet, daß**
die PRAPA eine zwischen 400 und 600 ms liegende und vorzugsweise 560 ms betragende Maximaldauer hat.

5. Verfahren gemäß Patentanspruch 1,
**dadurch gekennzeichnet, daß**
auf die Erfassung einer während der PRAPA auftretenden "P"-Zacke hin, ein aurikuläres Ausströmintervall ausgelöst wird, dessen Dauer gleich der PRAPA ist, und, in Abwesenheit der Erfassung einer späteren "P"-Zacke, eine aurikuläre Stimulation am Ende der längsten Verzögerung zwischen diesem letzten Intervall und dem anläßlich der vorhergehenden "P"-Zacke ausgelösten aurikulären Ausströmintervall ausgelöst wird.

6. Verfahren gemäß Patentanspruch 5,
**dadurch gekennzeichnet, daß**
im Fall der Erfassung einer "P"-Zacke außerhalb der PRAPA, oder im Fall der aurikulären Stimulation, eine aurikulär-ventrikuläre Verzögerung minimaler Dauer ausgelöst wird.

7. Verfahren gemäß Patentanspruch 5,
**dadurch gekennzeichnet, daß**
am Ende dieser minimalen aurikulär-ventrikulären Verzögerung eine ventrikuläre Stimulation ausgelöst wird, wenn die seit seiner letzten Stimulation vergangene Zeit es erlaubt, die maximale Frequenz der Stimulation zu berücksichtigen.

8. Verfahren gemäß Patentanspruch 5,
**dadurch gekennzeichnet, daß**
man, wenn man am Ende der minimalen aurikulär-ventrikulären Verzögerung diese verlängern muß, um die maximale Frequenz der Stimulation zu berücksichtigen, nach der ventrikulären Stimulation eine PRAPV auslöst, deren Dauer gleich der Zeit vor dem Trennen zweier Stimulationen mit maximaler Frequenz ist, reduziert um den Wert der minimalen aurikulär-ventrikulären Verzögerung.

9. Verfahren gemäß Patentanspruch 7 oder 8,
**dadurch gekennzeichnet, daß**
man nach dem Beobachten des Eintretens einer Tachykardie während einer definierten Zeit, vorzugsweise 30 Sekunden, das Absinken auslöst, das aus dem auf willkürliche Weise bestehenden Verringern der Frequenz der ventrikulären Stimulation auf eine beispielsweise auf 70 c/mn festgelegte stabile Frequenz besteht.

10. Verfahren gemäß Patentanspruch 9,
**dadurch gekennzeichnet, daß**
wenn im Verlauf der Phase des Absinkens das minimale Intervall über 8 Zyklen zwischen zwei aufeinanderfolgenden "P"-Zacken größer als die der programmierten maximalen Frequenz der ventrikulären Stimulation entsprechende minimale Periode Pmin wird, die Phase des Absinkens verlassen wird, und in die anfängliche Betriebsart der Stimulation des Herzens zurückgekehrt wird.

FIG.1

FIG.2

FIG.3

PRAPV = Pmin - DAVmin